# EUROPEAN PATENT APPLICATION

(11) **EP 4 643 786 A1**
(43) Date of publication of application: **05.11.2025**
(21) Application number: 24749907.2
(22) Date of filing: 12.01.2024
(51) Int. Cl.: A61B 8/12, A61B 1/00

(54) **CATHETER FOR DIAGNOSTIC IMAGING**

(30) Priority: 30.01.2023 JP 2023012265
(71) Applicant: Terumo Kabushiki Kaisha, Tokyo 151-0072 (JP)
(72) Inventor: TANI, Kentaro, Ashigarakami-gun, Kanagawa 2590151 (JP); KATO, Chihiro, Ashigarakami-gun, Kanagawa 2590151 (JP)
(74) Representative: Casalonga
(86) International application number: PCT/JP2024/000677
(87) International publication number: WO 2024/161952

(57) **Abstract**

This image diagnosis catheter has an outer tube, an inner tube, an inner-tube support tube, and a drive shaft in this order, and further includes: an inner tube spacer that holds the inner-tube support tube so as to define an inner-tube support tube outer flow path; a connector hub movable to a distal end side and a proximal end side relative to the outer tube together with the inner tube, the inner-tube support tube, the drive shaft, and the inner tube spacer; and an inner flow path and an outer flow path through which a priming solution flows. An inner-tube-spacer proximal-side flow path is formed on the proximal end side relative to the inner tube spacer. At least the inner tube spacer defines an inner-tube-spacer partition flow path. The inner flow path includes the inner-tube-spacer proximal-side flow path and an inner-tube support tube inner flow path in this order from an upstream side to a downstream side. The outer flow path includes the inner-tube-spacer proximal-side flow path, the inner-tube-spacer partition flow path, and the inner-tube support tube outer flow path in this order from the upstream side to the downstream side.

## Description

### Technical Field

The present disclosure relates to an image diagnosis catheter.

### Background Art

An image diagnosis catheter has been known that includes an outer tube, an inner tube, and a drive shaft in this order from a radially outer side toward a radially inner side and that further includes a connector hub movable to the distal end side and the proximal end side relative to the outer tube together with the inner tube and the drive shaft (see, for example, Patent Literature 1).

### Citation List

### Patent Literature

Patent Literature 1: JP 4672188 B1

### Summary of Invention

### Technical Problem

When the drive shaft is buckled inside the inner tube during an operation of pushing the inner tube into the outer tube, a load concentrates on the buckled portion upon activation, and damage may occur. In addition, a lumen defined by the outer tube, the inner tube, the drive shaft, and the connector hub needs to be filled with a priming solution before use.

In view of this, an object of the present disclosure is to provide an image diagnosis catheter that can prevent the occurrence of buckling of a drive shaft inside an inner tube and that enables easy priming.

### Solution to Problem

One aspect of the present disclosure is as follows.
[1] An image diagnosis catheter that includes an outer tube, an inner tube, an inner-tube support tube, and a drive shaft in this order from a radially outer side to a radially inner side, the image diagnosis catheter further including:
   an inner tube spacer that holds the inner-tube support tube so as to define an inner-tube support tube outer flow path;
   a connector hub movable to a distal end side and a proximal end side relative to the outer tube together with the inner tube, the inner-tube support tube, the drive shaft, and the inner tube spacer;
   an inner flow path and an outer flow path through which a priming solution flows; and
   an inner-tube-spacer proximal-side flow path formed on the proximal end side relative to the inner tube spacer, wherein
   at least the inner tube spacer defines an inner-tube-spacer partition flow path,
   the inner flow path includes the inner-tube-spacer proximal-side flow path and an inner-tube support tube inner flow path in this order from an upstream side to a downstream side, and
   the outer flow path includes the inner-tube-spacer proximal-side flow path, the inner-tube-spacer partition flow path, and the inner-tube support tube outer flow path in this order from the upstream side to the downstream side.
[2] The image diagnosis catheter according to [1], wherein
   the connector hub has a first inner circumferential surface that defines the inner-tube-spacer proximal-side flow path, a second inner circumferential surface that is provided on the radially outer side relative to the first inner circumferential surface, and a transition surface that extends from the first inner circumferential surface to the second inner circumferential surface, and
   the inner tube spacer has a first outer facing surface that faces the transition surface, a second outer facing surface that faces the second inner circumferential surface, and a third outer facing surface that faces a proximal-side end face of the inner tube.
[3] The image diagnosis catheter according to [2], wherein the inner tube spacer has a distal-side end face having the third outer facing surface.
[4] The image diagnosis catheter according to [2] or [3], wherein the inner tube spacer has a proximal-side end face having the first outer facing surface.
[5] The image diagnosis catheter according to any one of [2] to [4], wherein
   the transition surface and the first outer facing surface define a first flow path,
   the second inner circumferential surface and the second outer facing surface define a second flow path,
   the proximal-side end face of the inner tube and the third outer facing surface define a third flow path, and
   the inner-tube-spacer partition flow path includes the first flow path, the second flow path, and the third flow path in this order from the upstream side to the downstream side.
[6] The image diagnosis catheter according to [5], wherein
   the first outer facing surface has a recess that defines the first flow path,
   the second outer facing surface has a recess or a chamfered portion that defines the second flow path, and
   the third outer facing surface has a recess that defines the third flow path.
[7] The image diagnosis catheter according to any one of [1] to [6], wherein
   the inner tube spacer has a first inner facing surface that faces an outer circumferential surface of the inner-tube support tube and a second inner facing surface that faces a proximal-side end face of the inner-tube support tube.
[8] The image diagnosis catheter according to any one of [1] to [7], wherein
   the inner tube spacer has an adhesive introduction path extending inside the inner tube spacer from an inlet port opened in the second outer facing surface to an outer circumferential surface of the inner-tube support tube between the first outer facing surface and the third outer facing surface.
[9] The image diagnosis catheter according to any one of [1] to [8], wherein
   the second outer facing surface of the inner tube spacer is rotationally symmetric in a predetermined transverse plane perpendicular to a longitudinal direction of the inner tube.
[10] The image diagnosis catheter according to any one of [1] to [9], further including:
   an outer-tube support tube provided on the radially inner side with respect to the outer tube and on the radially outer side with respect to the drive shaft;
   an outer tube spacer that holds the outer-tube support tube so as to define an outer-tube support tube outer flow path;
   a relay connector movable to the distal end side and the proximal end side relative to the inner tube together with the outer tube, the outer-tube support tube, and the outer tube spacer; and
   an outer-tube-spacer distal-side flow path formed on the distal end side relative to the outer tube spacer, wherein
   at least the outer tube spacer defines an outer-tube-spacer partition flow path,
   the inner flow path includes an outer-tube support tube inner flow path and the outer-tube-spacer distal-side flow path in this order from the upstream side to the downstream side, and
   the outer flow path includes the outer-tube support tube outer flow path, the outer-tube-spacer partition flow path, and the outer-tube-spacer distal-side flow path in this order from the upstream side to the downstream side.
[11] The image diagnosis catheter according to [10], wherein
   the relay connector has a relay-connector first inner circumferential surface, a relay-connector second inner circumferential surface that is provided on the radially inner side with respect to the relay-connector first inner circumferential surface and that defines the outer-tube-spacer distal-side flow path, and a relay-connector transition surface that extends from the relay-connector first inner circumferential surface to the relay-connector second inner circumferential surface, and
   the outer tube spacer has a first facing surface that faces an inner circumferential surface of the outer tube at a distal end portion, a second facing surface that faces a distal-side end face of the outer tube, a third facing surface that faces the relay-connector first inner circumferential surface, and a fourth facing surface that faces the relay-connector transition surface.
[12] The image diagnosis catheter according to [11], wherein
   the inner circumferential surface of the outer tube at the distal end portion and the outer tube spacer define an outer-tube-spacer first flow path,
   the distal-side end face of the outer tube and the second facing surface define an outer-tube-spacer second flow path,
   the relay-connector first inner circumferential surface and the third facing surface define an outer-tube-spacer third flow path,
   the relay-connector transition surface and the fourth facing surface define an outer-tube-spacer fourth flow path, and
   the outer-tube-spacer partition flow path includes the outer-tube-spacer first flow path, the outer-tube-spacer second flow path, the outer-tube-spacer third flow path, and the outer-tube-spacer fourth flow path in this order from the upstream side to the downstream side.
[13] The image diagnosis catheter according to [12], wherein
   the first facing surface has a recess or a chamfered portion that defines the outer-tube-spacer first flow path,
   the second facing surface has a recess that defines the outer-tube-spacer second flow path,
   the third facing surface has a recess or a chamfered portion that defines the outer-tube-spacer third flow path, and
   the fourth facing surface has a recess that defines the outer-tube-spacer fourth flow path.
[14] The image diagnosis catheter according to any one of [10] to [13], wherein
   the outer-tube support tube is provided on the radially inner side with respect to the inner tube and on the radially outer side with respect to the inner-tube support tube, and
   in a most extended state in which the inner tube is maximally drawn out of the outer tube, a proximal-side end face of the outer-tube support tube is located distal to a distal-side end face of the inner tube, and a clearance is formed between an inner circumferential surface of the outer-tube support tube at a proximal end portion and an outer circumferential surface of the inner-tube support tube at a distal end portion.

### Advantageous Effects of Invention

The present disclosure can provide an image diagnosis catheter that can prevent the occurrence of buckling of a drive shaft inside an inner tube and that enables easy priming.

### Brief Description of Drawings

Fig. 1 is an external view illustrating the most contracted state of an image diagnosis catheter according to an embodiment.
Fig. 2 is an external view illustrating the most extended state of the image diagnosis catheter illustrated in Fig. 1.
Fig. 3 is a cross-sectional view illustrating a telescope portion in the state illustrated in Fig. 2.
Fig. 4 is a perspective view of an inner tube spacer illustrated in Fig. 3.
Fig. 5 is a perspective view from an angle different from that in Fig. 4.
Fig. 6 is a perspective view illustrating a first modification of the inner tube spacer.
Fig. 7 is a perspective view from an angle different from that in Fig. 6.
Fig. 8 is a perspective view illustrating a second modification of the inner tube spacer.
Fig. 9 is a perspective view from an angle different from that in Fig. 8.
Fig. 10 is a perspective view illustrating a third modification of the inner tube spacer.
Fig. 11 is a perspective view from an angle different from that in Fig. 10.
Fig. 12 is a perspective view from an angle different from the angles in Figs. 10 and 11.
Fig. 13 is a perspective view illustrating a modification of an outer tube spacer.
Fig. 14 is a plan view from an angle different from that in Fig. 13.
Fig. 15 is a cross-sectional view taken along line A-A in Fig. 14.

### Description of Embodiments

An embodiment of the present disclosure will be described in detail below with reference to the drawings.

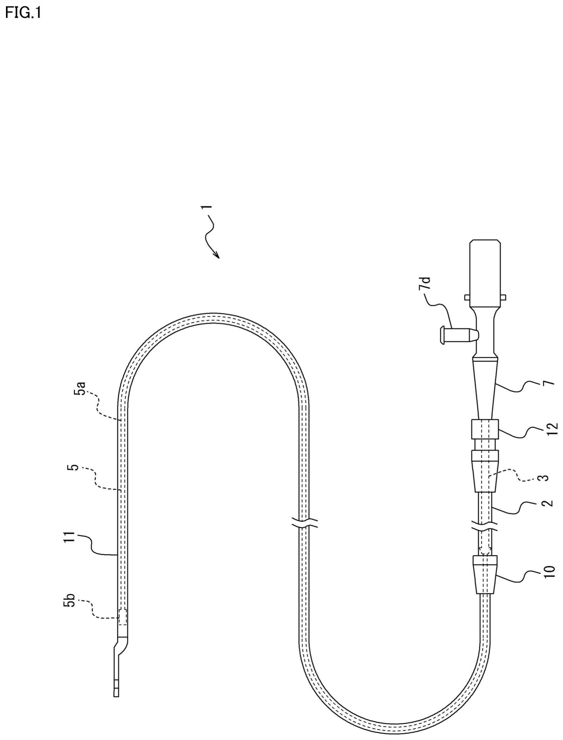

An image diagnosis catheter 1 according to an embodiment of the present disclosure illustrated in Figs. 1 to 5 includes an outer tube 2, an inner tube 3, an inner-tube support tube 4, and a drive shaft 5a in this order from a radially outer side to a radially inner side. The image diagnosis catheter 1 further includes an inner tube spacer 6 that holds the inner-tube support tube 4 so as to define an inner-tube support tube outer flow path P3, a connector hub 7 that is movable to the distal end side and the proximal end side relative to the outer tube 2 together with the inner tube 3, the inner-tube support tube 4, the drive shaft 5a, and the inner tube spacer 6, and an inner flow path Pi and an outer flow path Po through which a priming solution (for example, physiological saline) flows. An inner-tube-spacer proximal-side flow path P1 is formed on the proximal end side relative to the inner tube spacer 6. At least the inner tube spacer 6 defines an inner-tube-spacer partition flow path P2. The inner flow path Pi includes the inner-tube-spacer proximal-side flow path P1 and an inner-tube support tube inner flow path P7 in this order from the upstream side to the downstream side. The outer flow path Po includes the inner-tube-spacer proximal-side flow path P1, the inner-tube-spacer partition flow path P2, and the inner-tube support tube outer flow path P3 in this order from the upstream side to the downstream side.

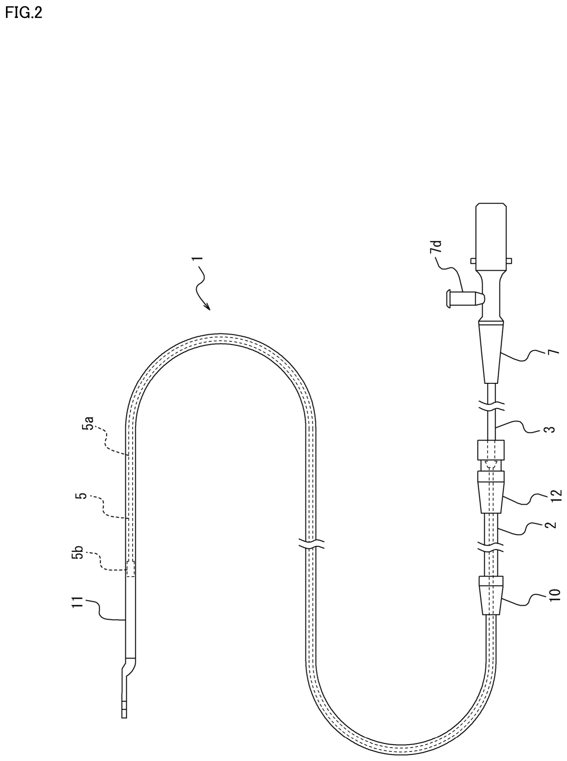

According to the above configuration, the inner-tube support tube 4 can prevent the drive shaft 5a from being buckled in the inner tube 3 during the pushing operation (for example, an operation to change the state illustrated in Fig. 2 to the state illustrated in Fig. 1) of pushing the inner tube 3 into the outer tube 2. In addition, the inner tube spacer 6 not only holds the inner-tube support tube 4 but also can appropriately partition the lumen into the inner flow path Pi and the outer flow path Po, so that easy priming is enabled.

The connector hub 7 includes a first inner circumferential surface 7a that defines the inner-tube-spacer proximal-side flow path P1, a second inner circumferential surface 7c provided radially outside the first inner circumferential surface 7a, and a transition surface 7b extending from the first inner circumferential surface 7a to the second inner circumferential surface 7c. The inner tube spacer 6 includes a first outer facing surface 6a facing the transition surface 7b, a second outer facing surface 6b facing the second inner circumferential surface 7c, and a third outer facing surface 6c facing the proximal-side end face of the inner tube 3. According to the above configuration, the inner tube spacer 6 and the inner-tube support tube 4 can be satisfactorily positioned with respect to the connector hub 7 and the inner tube 3 due to the contact between the transition surface 7b and the first outer facing surface 6a and the contact between the proximal-side end face of the inner tube 3 and the third outer facing surface 6c.

A distal-side end face 6d of the inner tube spacer 6 has the third outer facing surface 6c. According to the above configuration, the structure of the inner tube spacer 6 can be simplified.

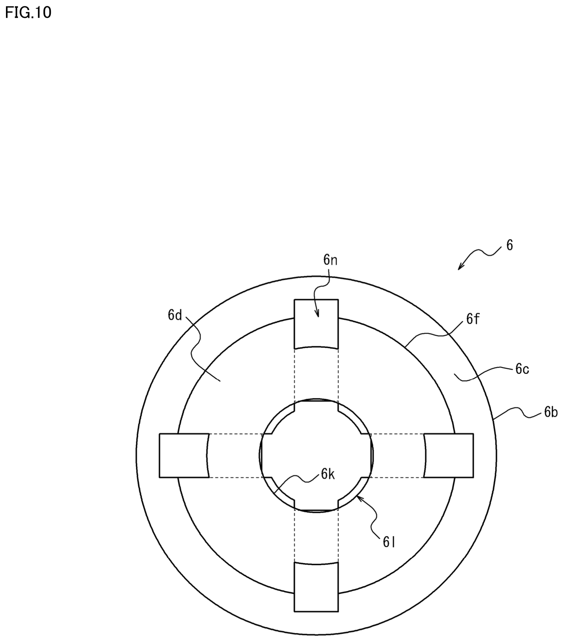

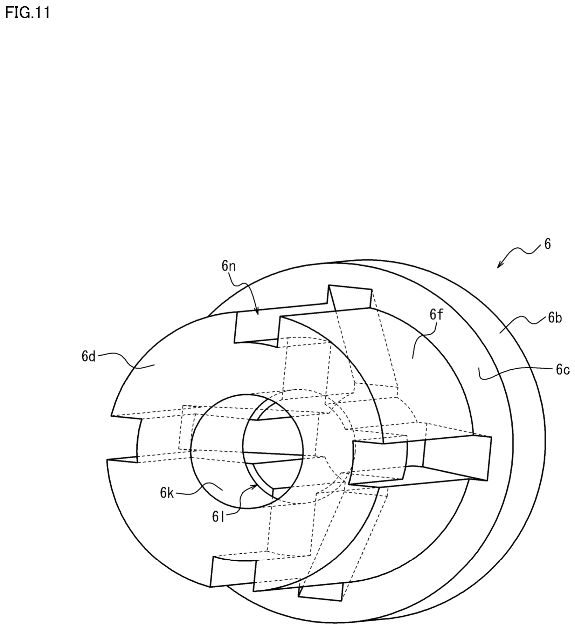

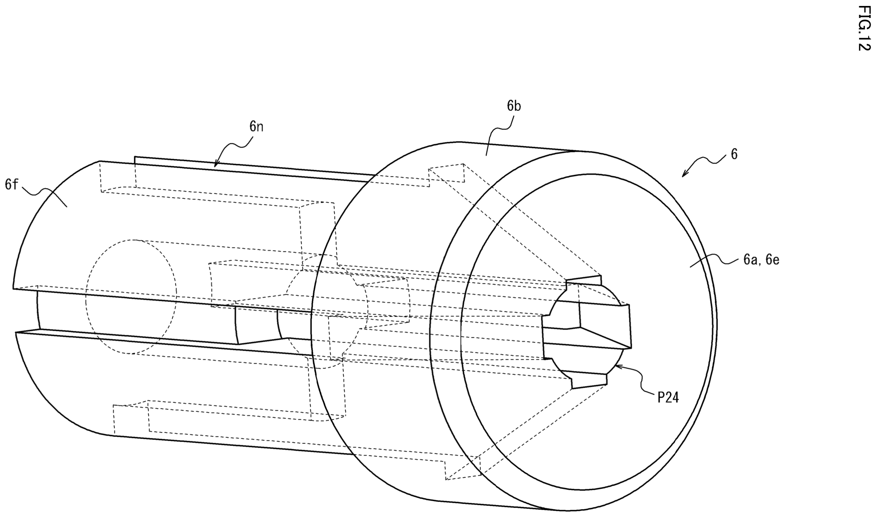

As in a third modification illustrated in Figs. 10 to 12, the distal-side end face 6d of the inner tube spacer 6 may be provided distal to the third outer facing surface 6c. The inner tube spacer 6 according to the third modification has a fourth outer facing surface 6f facing the inner circumferential surface of the inner tube 3 at a proximal end portion between the distal-side end face 6d of the inner tube spacer 6 and the third outer facing surface 6c. According to the above configuration, force for holding the inner-tube support tube 4 can be increased by the contact between the inner circumferential surface of the inner tube 3 at the proximal end portion and the fourth outer facing surface 6f.

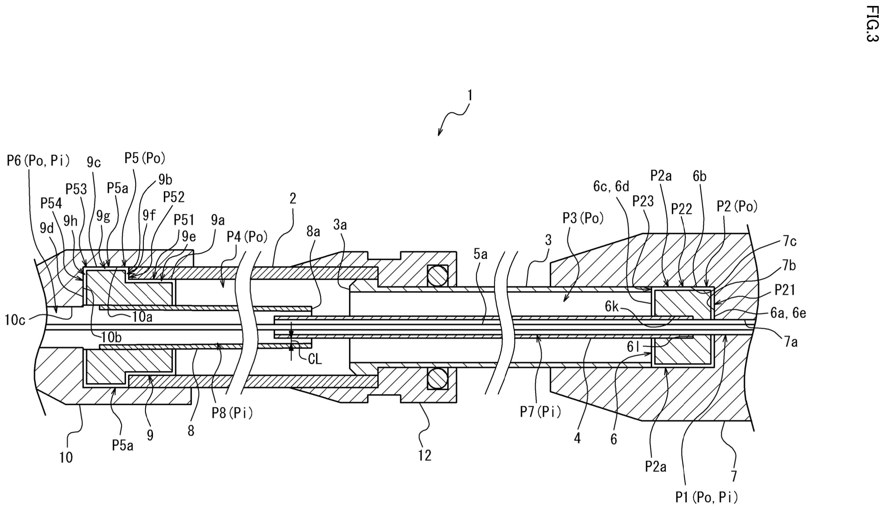

As illustrated in Figs. 3 to 5, a proximal-side end face 6e of the inner tube spacer 6 has the first outer facing surface 6a. According to the above configuration, the structure of the inner tube spacer 6 can be simplified.

The transition surface 7b and the first outer facing surface 6a define a first flow path P21. The second inner circumferential surface 7c and the second outer facing surface 6b define a second flow path P22. The proximal-side end face of the inner tube 3 and the third outer facing surface 6c define a third flow path P23. The inner-tube-spacer partition flow path P2 includes the first flow path P21, the second flow path P22, and the third flow path P23 in this order from the upstream side to the downstream side. According to the above configuration, the inner-tube-spacer partition flow path P2 can be easily provided to have a sufficient length with a small cross-sectional area, and the direction of the flow path can be changed from the first flow path P21 to the second flow path P22 and from the second flow path P22 to the third flow path P23. Therefore, the flow path resistance of the outer flow path Po can be easily increased by the inner-tube-spacer partition flow path P2.

As the inner-tube support tube 4 is set to be thinner in order to easily prevent the drive shaft 5a from being buckled, the flow path resistance of the inner flow path Pi increases and the flow path resistance of the outer flow path Po decreases, so that the priming solution flowing through the outer flow path Po is likely to precede during priming. In this case, the priming solution flowing through the outer flow path Po enters the inner flow path Pi through the junction between the inner flow path Pi and the outer flow path Po, flows backward to the proximal end side, and traps air with the priming solution flowing later through the inner flow path Pi, and the trapped air remains in the lumen. However, by increasing the flow path resistance of the outer flow path Po by the inner-tube-spacer partition flow path P2 as described above, the timing at which the priming solution flowing through the outer flow path Po travels from the proximal end side to the distal end side and the timing at which the priming solution flowing through the inner flow path Pi travels from the proximal end side to the distal end side can be approximately synchronized with each other in an easy way. Therefore, both the prevention of buckling and easy priming with less number of times of priming can be efficiently achieved.

As in the third modification, the inner tube spacer 6 may have an internal flow path P24 provided so as to pass through the inside of the inner tube spacer 6 from the first outer facing surface 6a to the third outer facing surface 6c instead of the first flow path P21, the second flow path P22, and the third flow path P23. According to the above configuration, the inner-tube-spacer partition flow path P2 can be easily implemented by the internal flow path P24.

In the third modification, the inner circumferential surface of the inner tube 3 at the proximal end portion and the fourth outer facing surface 6f define a fourth flow path, and the inner-tube-spacer partition flow path P2 includes the internal flow path P24 and the fourth flow path in this order from the upstream side to the downstream side. According to the above configuration, the flow path resistance of the outer flow path Po can be easily increased by the inner-tube-spacer partition flow path P2.

In the third modification, the fourth outer facing surface 6f has a recess (fourth recess 6n) that defines the fourth flow path. According to the above configuration, the fourth flow path can be easily set with the shape of the inner tube spacer 6.

The inner-tube-spacer partition flow path P2 may include the first flow path P21, the second flow path P22, the third flow path P23, and the fourth flow path in this order from the upstream side to the downstream side (not illustrated). According to the above configuration, the flow path resistance of the outer flow path Po can also be easily increased by the inner-tube-spacer partition flow path P2.

As illustrated in Figs. 3 to 5, the first outer facing surface 6a has a recess (first recess 6g) defining the first flow path P21, the second outer facing surface 6b has a chamfered portion 6h defining the second flow path P22, and the third outer facing surface 6c has a recess (third recess 6i) defining the third flow path P23. According to the above configuration, the inner-tube-spacer partition flow path P2 can be easily set with the shape of the inner tube spacer 6.

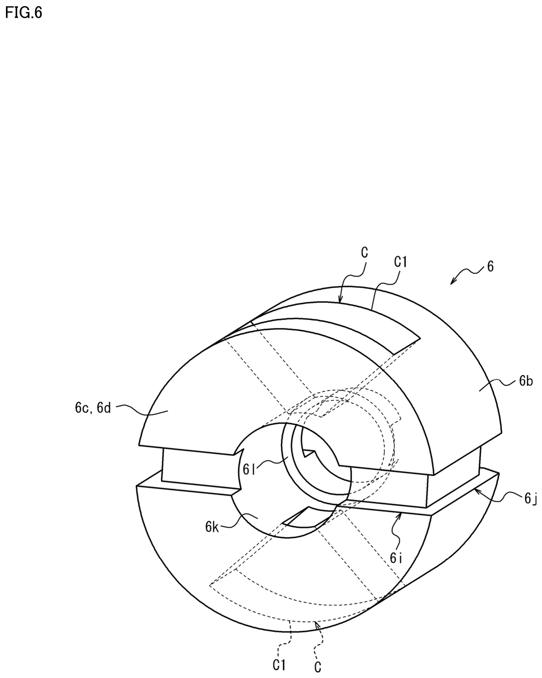

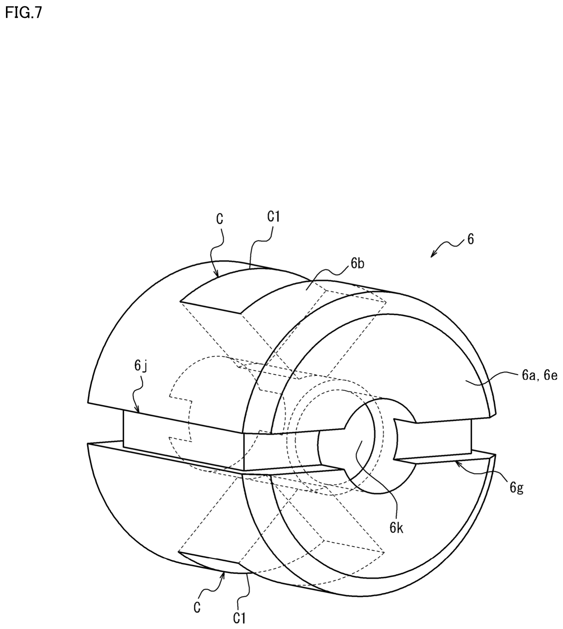

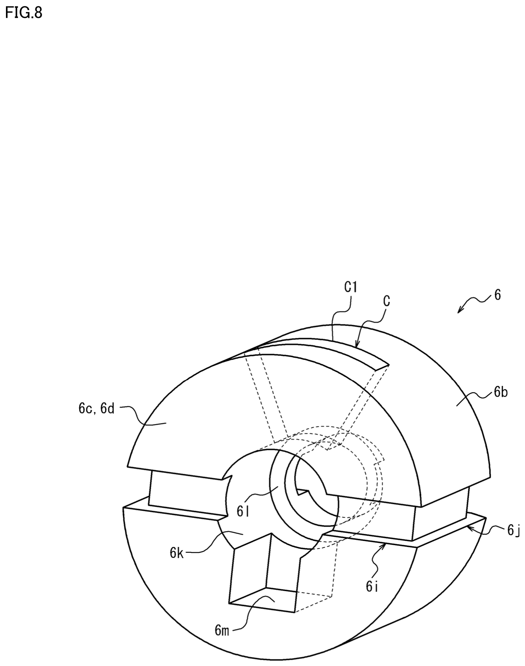

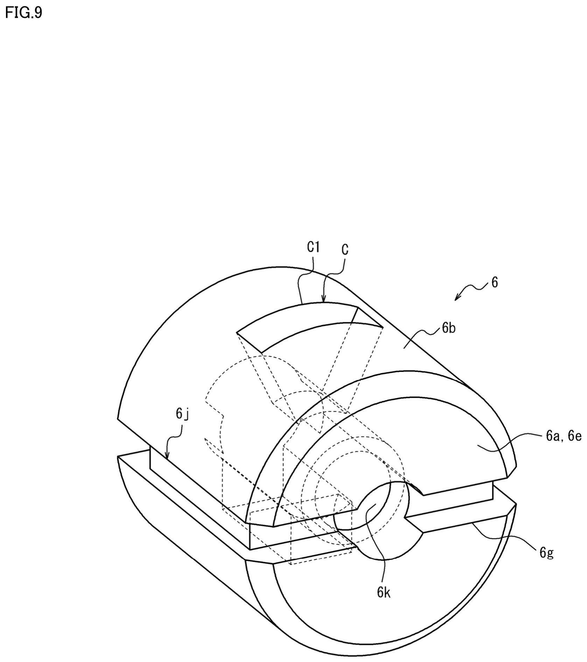

As in a first modification illustrated in Figs. 6 and 7 and a second modification illustrated in Figs. 8 and 9, the first outer facing surface 6a may have a recess (first recess 6g) that defines the first flow path P21, the second outer facing surface 6b may have a recess (second recess 6j) that defines the second flow path P22, and the third outer facing surface 6c may have a recess (third recess 6i) that defines the third flow path P23. According to the above configuration, the inner-tube-spacer partition flow path P2 can also be easily set with the shape of the inner tube spacer 6.

As illustrated in Figs. 3 to 5, the inner tube spacer 6 has a first inner facing surface 6k facing the outer circumferential surface of the inner-tube support tube 4 and a second inner facing surface 6l facing the proximal-side end face of the inner-tube support tube 4. According to the above configuration, the inner-tube support tube 4 can be satisfactorily positioned due to the contact between the proximal-side end face of the inner-tube support tube 4 and the second inner facing surface 6l.

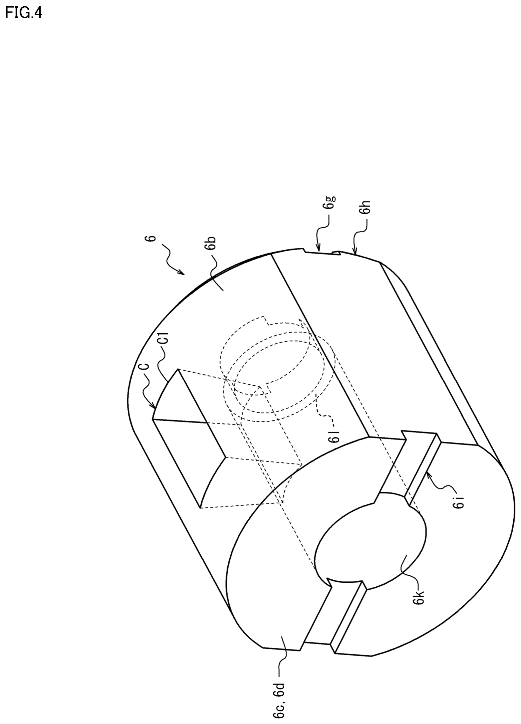

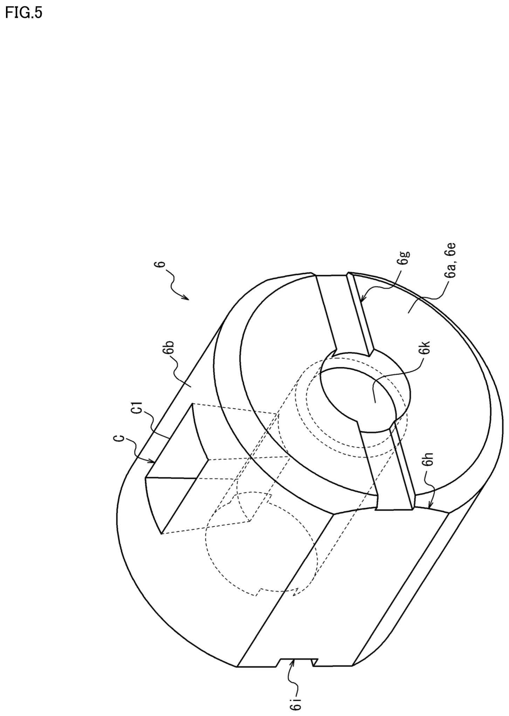

As illustrated in Figs. 4 and 5, the inner tube spacer 6 has an adhesive introduction path C extending inside the inner tube spacer 6 from an inlet port C1 opened in the second outer facing surface 6b to the outer circumferential surface of the inner-tube support tube 4 between the first outer facing surface 6a and the third outer facing surface 6c. According to the above configuration, the inner-tube support tube 4 can be easily fixed to the inner tube spacer 6 by introducing a liquid adhesive from the inlet port C1 into the adhesive introduction path C and solidifying the adhesive. In addition, the use of the adhesive introduction path C can prevent the adhesive from protruding to the outer flow path Po or from being present in a planned contact portion (for example, between the proximal-side end face of the inner tube 3 and the third outer facing surface 6c) for positioning the inner tube spacer 6. Therefore, the shape of the inner-tube support tube outer flow path P3 designed to achieve satisfactory priming can be accurately obtained in a manufacturing process.

As indicated in the first modification, the inner tube spacer 6 may have a plurality of adhesive introduction paths C. As indicated in the second modification, the inner tube spacer 6 may have a recessed adhesive reservoir 6m that stores the adhesive introduced from the adhesive introduction path C in the first inner facing surface 6k. As indicated in the third modification, the inner tube spacer 6 may not have the adhesive introduction path C.

As illustrated in Figs. 4 and 5, the second outer facing surface 6b of the inner tube spacer 6 is rotationally symmetric in a predetermined transverse plane perpendicular to the longitudinal direction of the inner tube 3. According to the above configuration, the contact between the second inner circumferential surface 7c and the rotationally symmetric second outer facing surface 6b can prevent the misalignment of the inner tube spacer 6 and the inner-tube support tube 4 with respect to the second inner circumferential surface 7c of the connector hub 7. In addition, the contact between the second inner circumferential surface 7c of the connector hub 7 and the outer circumferential surface of the inner tube 3 can prevent the misalignment of the inner tube spacer 6 and the inner-tube support tube 4 with respect to the inner tube 3 that is aligned with respect to the second inner circumferential surface 7c of the connector hub 7. In addition, even if misalignment of the inner tube spacer 6 occurs, the influence of the misalignment on the cross-sectional area of the inner-tube-spacer partition flow path P2 (second flow path P22) can be reduced by the rotationally symmetric structure of the second outer facing surface 6b. Therefore, the ease of priming can be enhanced by accurately obtaining the designed shapes of the outer flow path Po and inner flow path Pi. The second inner circumferential surface 7c and the rotationally symmetric second outer facing surface 6b may not contact each other.

As illustrated in Figs. 3 to 5, the inner-tube-spacer partition flow path P2 has a plurality of branched flow paths P2a that branch so as to be rotationally symmetric with respect to the central axis of the inner tube spacer 6. According to the above configuration, the influence of the misalignment on the cross-sectional area of the inner-tube-spacer partition flow path P2 (second flow path P22) can be reduced, whereby it is possible to enhance the ease of priming.

As illustrated in Fig. 3, the image diagnosis catheter 1 further includes an outer-tube support tube 8 provided on the radially inner side with respect to the outer tube 2 and on the radially outer side with respect to the drive shaft 5a, an outer tube spacer 9 that holds the outer-tube support tube 8 so as to define an outer-tube support tube outer flow path P4, and a relay connector 10 that is movable to the distal end side and the proximal end side relative to the inner tube 3 together with the outer tube 2, the outer-tube support tube 8, and the outer tube spacer 9. An outer-tube-spacer distal-side flow path P6 is formed on the distal end side relative to the outer tube spacer 9. At least the outer tube spacer 9 defines an outer-tube-spacer partition flow path P5. The inner flow path Pi includes the outer-tube support tube inner flow path P8 and the outer-tube-spacer distal-side flow path P6 in this order from the upstream side to the downstream side. The outer flow path Po includes the outer-tube support tube outer flow path P4, the outer-tube-spacer partition flow path P5, and the outer-tube-spacer distal-side flow path P6 in this order from the upstream side to the downstream side. According to the above configuration, the outer-tube support tube 8 can prevent the drive shaft 5a from being buckled in the outer tube 2 during the pushing operation of pushing the inner tube 3 into the outer tube 2. In addition, the outer tube spacer 9 not only holds the outer-tube support tube 8 but also can appropriately partition the lumen into the inner flow path Pi and the outer flow path Po, so that the ease of priming can be enhanced.

The relay connector 10 includes a relay-connector first inner circumferential surface 10a, a relay-connector second inner circumferential surface 10c that is provided radially inside the relay-connector first inner circumferential surface 10a and defines the outer-tube-spacer distal-side flow path P6, and a relay-connector transition surface 10b extending from the relay-connector first inner circumferential surface 10a to the relay-connector second inner circumferential surface 10c. The outer tube spacer 9 includes a first facing surface 9a facing the inner circumferential surface of the outer tube 2 at the distal end portion, a second facing surface 9b facing a distal-side end face of the outer tube 2, a third facing surface 9c facing the relay-connector first inner circumferential surface 10a, and a fourth facing surface 9d facing the relay-connector transition surface 10b. According to the above configuration, the outer tube spacer 9 and the outer-tube support tube 8 can be satisfactorily positioned with respect to the relay connector 10 and the outer tube 2 due to the contact between the inner circumferential surface of the outer tube 2 at the distal end portion and the first facing surface 9a, the contact between the distal-side end face of the outer tube 2 and the second facing surface 9b, and the contact between the relay-connector transition surface 10b and the fourth facing surface 9d.

The inner circumferential surface of the outer tube 2 at the distal end portion and the outer tube spacer 9 define an outer-tube-spacer first flow path P51. The distal-side end face of the outer tube 2 and the second facing surface 9b define an outer-tube-spacer second flow path P52. The relay-connector first inner circumferential surface 10a and the third facing surface 9c define an outer-tube-spacer third flow path P53. The relay-connector transition surface 10b and the fourth facing surface 9d define an outer-tube-spacer fourth flow path P54. The outer-tube-spacer partition flow path P5 includes the outer-tube-spacer first flow path P51, the outer-tube-spacer second flow path P52, the outer-tube-spacer 3 flow path, and the outer-tube-spacer fourth flow path P54 in this order from the upstream side to the downstream side. According to the above configuration, the outer-tube-spacer partition flow path P5 can be easily provided to have a sufficient length with a small cross-sectional area, whereby the flow path resistance of the outer flow path Po can be easily increased by the outer-tube-spacer partition flow path P5.

The inner circumferential surface of the outer tube 2 at the distal end portion and the first facing surface 9a define the outer-tube-spacer first flow path P51. According to the above configuration, the cross-sectional area of the outer-tube-spacer first flow path P51 can be easily decreased, whereby the flow path resistance of the outer-tube-spacer first flow path P51 can be easily increased.

The second facing surface 9b has a recess (outer-tube-spacer second recess 9f) that defines the outer-tube-spacer second flow path P52. The third facing surface 9c has a recess (outer-tube-spacer third recess 9g) that defines the outer-tube-spacer third flow path P53. The fourth facing surface 9d has a recess (outer-tube-spacer fourth recess 9h) that defines the outer-tube-spacer fourth flow path P54. According to the above configuration, the outer-tube-spacer partition flow path P5 can be easily set with the shape of the outer tube spacer 9. The third facing surface 9c may have a chamfered portion that defines the outer-tube-spacer third flow path P53 instead of the outer-tube-spacer third recess 9g.

The first facing surface 9a has a recess (outer-tube-spacer first recess 9e) that defines the outer-tube-spacer first flow path P51. According to the above configuration, the outer-tube-spacer partition flow path P5 can be easily set with the shape of the outer tube spacer 9. The first facing surface 9a may have a chamfered portion that defines the outer-tube-spacer first flow path P51 instead of the outer-tube-spacer first recess 9e.

As illustrated in Fig. 3, the outer-tube-spacer partition flow path P5 has a plurality of outer-tube-spacer partition branch flow paths P5a that branches so as to be rotationally symmetric with respect to the central axis of the outer tube spacer 9. According to the above configuration, the influence of the misalignment of the outer tube spacer 9 on the cross-sectional area of the outer-tube-spacer partition flow path P5 (outer-tube-spacer first flow path P51, outer-tube-spacer third flow path P53) can be reduced, whereby it is possible to enhance the ease of priming.

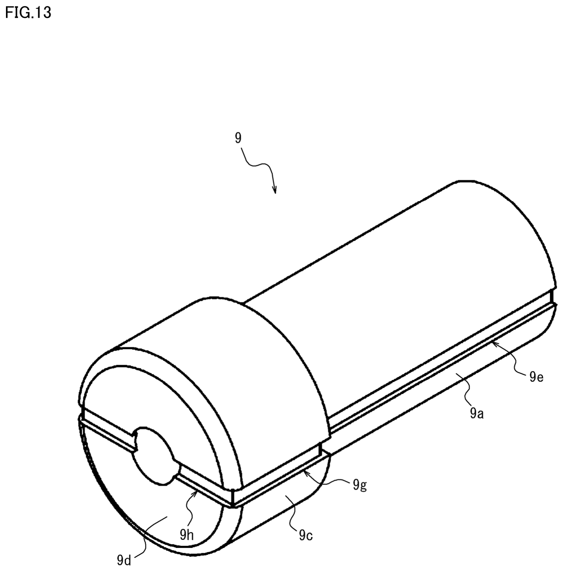

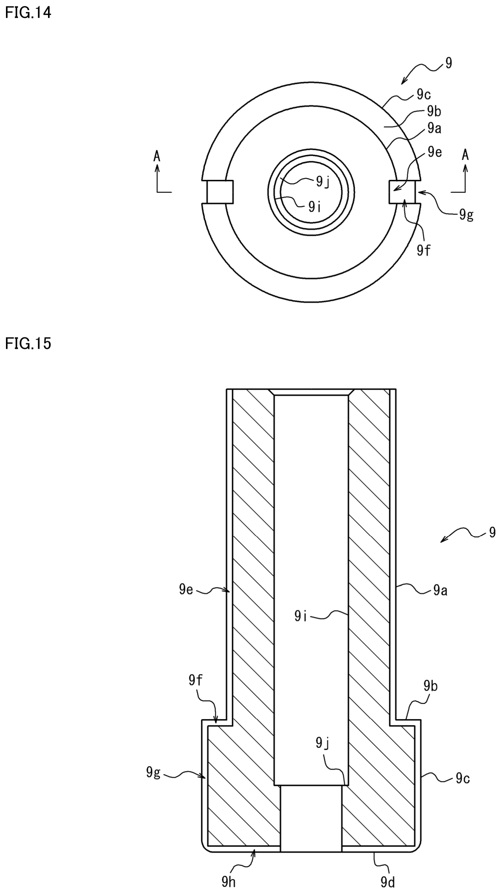

As in a modification illustrated in Figs. 13 to 15, the outer tube spacer 9 may have an outer-tube-spacer first inner facing surface 9i facing the outer circumferential surface of the outer-tube support tube 8 and an outer-tube-spacer second inner facing surface 9j facing the distal-side end face of the outer-tube support tube 8. According to the above configuration, the outer-tube support tube 8 can be satisfactorily positioned due to the contact between the distal-side end face of the outer-tube support tube 8 and the outer-tube-spacer second inner facing surface 9j.

The outer-tube support tube 8 is provided radially inside the inner tube 3 and radially outside the inner-tube support tube 4. In the most extended state in which the inner tube 3 is maximally drawn out of the outer tube 2, a proximal-side end face 8a of the outer-tube support tube 8 is located distal to a distal-side end face 3a of the inner tube 3, so that a clearance CL is formed between the inner circumferential surface of the outer-tube support tube 8 at the proximal end portion and the outer circumferential surface of the inner-tube support tube 4 at the distal end portion. According to the above configuration, it is possible to prevent a change in the cross-sectional area of the outer flow path Po at a transition portion from the inside of the inner tube 3 to the inside of the outer tube 2 when priming is performed in the most extended state, whereby it is possible to prevent the priming solution from trapping air in the transition portion. Therefore, the ease of priming can be enhanced. In addition, by appropriately setting the clearance CL, an appropriate amount of priming solution can flow from the inner-tube support tube outer flow path P3 to the outer-tube support tube inner flow path P8. This configuration makes it easy to synchronize the timing of flow of the priming solution through the outer flow path Po and the timing of flow of the priming solution through the inner flow path Pi, and thus, the ease of priming can be improved.

The image diagnosis catheter 1 acquires a tomographic image of a living body using intravascular ultrasound (IVUS) and/or optical coherence tomography (OCT). According to the above configuration, the tomographic image of the living body can be easily acquired.

As illustrated in Figs. 1 and 2, the image diagnosis catheter 1 includes a sheath 11 to be inserted into a body cavity such as a vascular channel of a living body, the outer tube 2 connected to a proximal end of the sheath 11, the inner tube 3 inserted into the outer tube 2 and movable forward and backward, a unit connector 12 that is connected to the proximal end of the outer tube 2 and holds the inner tube 3 while allowing the inner tube 3 to move forward and backward, and the connector hub 7 connected to the proximal end of the inner tube 3. The image diagnosis catheter 1 also includes the drive shaft 5a and an imaging core 5 that is provided at the distal end portion of the drive shaft 5a and that includes a signal transmitter and receiver 5b transmitting and receiving a signal (ultrasound wave and/or light). The imaging core 5 is inserted into the sheath 11, the outer tube 2, and the inner tube 3 and axially movable forward and backward with the inner tube 3 relative to the sheath 11 and the outer tube 2. For example, when an operation of pushing the connector hub 7 toward the distal end side, that is, a push-in operation, is performed for changing the state illustrated in Fig. 2 to the state illustrated in Fig. 1, the imaging core 5 advances inside the sheath 11, that is, moves to the distal end side. For example, when an operation of pulling the connector hub 7 toward the proximal end side, that is, a pull-back operation, is performed for changing the state illustrated in Fig. 1 to the state illustrated in Fig. 2, the imaging core 5 moves to the proximal end side inside the sheath 11. At diagnosis, the sheath 11 is inserted into a body cavity and the imaging core 5, which is driven by an external device (not illustrated) to rotate at a rotational speed of about 1000 to 10000 rpm, moves backward inside the lumen of the sheath 11 due to the pull-back operation by the external device. At this time, the external device causes the signal transmitter and receiver 5b to transmit and receive a signal. An image indicating the state of a tissue around the body cavity is generated based on the signal received by scanning operation performed by rotating and moving the imaging core 5 backward. According to the above configuration, the image diagnosis catheter 1 using IVUS and/or OCT can be easily configured.

The distal end portion of the sheath 11 has a priming solution discharge port (not illustrated) that allows the lumen of the sheath 11 to communicate with the outside. The connector hub 7 has a port 7d serving as an inlet of a flow path for introducing the priming solution to the inner-tube-spacer proximal-side flow path P1. Priming is performed as follows. Specifically, an injection device (not illustrated) such as a syringe is attached to the port 7d, and the priming solution is introduced from the injection device, is passed through the inner flow path Pi and the outer flow path Po, and is discharged through the priming solution discharge port. According to the above configuration, the ease of priming can be enhanced.

The present disclosure is not limited to the above-described embodiment and may be modified in various manners without departing from the gist of the present disclosure.

Therefore, the image diagnosis catheter 1 according to the above-described embodiment can be variously modified as long as it has an outer tube 2, an inner tube 3, an inner-tube support tube 4, and a drive shaft 5a in this order from the radially outer side to the radially inner side and includes: an inner tube spacer 6 that holds the inner-tube support tube 4 so as to define an inner-tube support tube outer flow path P3; a connector hub 7 movable to the distal end side and the proximal end side relative to the outer tube 2 together with the inner tube 3, the inner-tube support tube 4, the drive shaft 5a, and the inner tube spacer 6; an inner flow path Pi and an outer flow path Po through which a priming solution flows; and an inner-tube-spacer proximal-side flow path P1 formed on the proximal end side relative to the inner tube spacer 6, wherein at least the inner tube spacer 6 defines an inner-tube-spacer partition flow path P2, the inner flow path Pi includes the inner-tube-spacer proximal-side flow path P1 and an inner-tube support tube inner flow path P7 in this order from an upstream side to a downstream side, and the outer flow path Po includes the inner-tube-spacer proximal-side flow path P1, the inner-tube-spacer partition flow path P2, and the inner-tube support tube outer flow path P3 in this order from the upstream side to the downstream side.

For example, the inner-tube-spacer partition flow path P2 may be defined by a recess provided in the connector hub 7 and a notch provided in the inner tube 3 instead of or in addition to the recess or the chamfered portion provided in the inner tube spacer 6. The outer-tube-spacer partition flow path P5 may be defined by a recess provided in the relay connector 10 and a recess provided in the outer tube 2 instead of or in addition to the recess or the chamfered portion provided in the outer tube spacer 9.

### Reference Signs List

- 1: image diagnosis catheter
- 2: outer tube
- 3: inner tube
- 3a: distal-side end face
- 4: inner-tube support tube
- 5: imaging core
- 5a: drive shaft
- 5b: signal transmitter and receiver
- 6: inner tube spacer
- 6a: first outer facing surface
- 6b: second outer facing surface
- 6c: third outer facing surface
- 6d: distal-side end face
- 6e: proximal-side end face
- 6f: fourth outer facing surface
- 6g: first recess
- 6h: chamfered portion
- 6i: third recess
- 6j: second recess
- 6k: first inner facing surface
- 6l: second inner facing surface
- 6m: adhesive reservoir
- 6n: fourth recess
- 7: connector hub
- 7a: first inner circumferential surface
- 7b: transition surface
- 7c: second inner circumferential surface
- 7d: port
- 8: outer-tube support tube
- 8a: proximal-side end face
- 9: outer tube spacer
- 9a: first facing surface
- 9b: second facing surface
- 9c: third facing surface
- 9d: fourth facing surface
- 9e: outer-tube-spacer first recess
- 9f: outer-tube-spacer second recess
- 9g: outer-tube-spacer third recess
- 9h: outer-tube-spacer fourth recess
- 9i: outer-tube-spacer first inner facing surface
- 9j: outer-tube-spacer second inner facing surface
- 10: relay connector
- 10a: relay-connector first inner circumferential surface
- 10b: relay-connector transition surface
- 10c: relay-connector second inner circumferential surface
- 11: sheath
- 12: unit connector
- C: adhesive introduction path
- C1: inlet port
- CL: clearance
- Pi: inner flow path
- Po: outer flow path
- P1: inner-tube-spacer proximal-side flow path
- P2: inner-tube-spacer partition flow path
- P21: first flow path
- P22: second flow path
- P23: third flow path
- P24: internal flow path
- P2a: branch flow path
- P3: inner-tube support tube outer flow path
- P4: outer-tube support tube outer flow path
- P5: outer-tube-spacer partition flow path
- P51: outer-tube-spacer first flow path
- P52: outer-tube-spacer second flow path
- P53: outer-tube-spacer third flow path
- P54: outer-tube-spacer fourth flow path
- P5a: outer-tube-spacer partition branch flow path
- P6: outer-tube-spacer distal-side flow path
- P7: inner-tube support tube inner flow path
- P8: outer-tube support tube inner flow path

## Claims

1. An image diagnosis catheter that includes an outer tube, an inner tube, an inner-tube support tube, and a drive shaft in this order from a radially outer side to a radially inner side, the image diagnosis catheter further comprising:
an inner tube spacer that holds the inner-tube support tube so as to define an inner-tube support tube outer flow path;
a connector hub movable to a distal end side and a proximal end side relative to the outer tube together with the inner tube, the inner-tube support tube, the drive shaft, and the inner tube spacer;
an inner flow path and an outer flow path through which a priming solution flows; and
an inner-tube-spacer proximal-side flow path formed on the proximal end side relative to the inner tube spacer,
wherein
at least the inner tube spacer defines an inner-tube-spacer partition flow path,
the inner flow path includes the inner-tube-spacer proximal-side flow path and an inner-tube support tube inner flow path in this order from an upstream side to a downstream side, and
the outer flow path includes the inner-tube-spacer proximal-side flow path, the inner-tube-spacer partition flow path, and the inner-tube support tube outer flow path in this order from the upstream side to the downstream side.

2. The image diagnosis catheter according to claim 1, wherein
the connector hub has a first inner circumferential surface that defines the inner-tube-spacer proximal-side flow path, a second inner circumferential surface that is provided on the radially outer side relative to the first inner circumferential surface, and a transition surface that extends from the first inner circumferential surface to the second inner circumferential surface, and
the inner tube spacer has a first outer facing surface that faces the transition surface, a second outer facing surface that faces the second inner circumferential surface, and a third outer facing surface that faces a proximal-side end face of the inner tube.

3. The image diagnosis catheter according to claim 2, wherein the inner tube spacer has a distal-side end face having the third outer facing surface.

4. The image diagnosis catheter according to claim 2, wherein the inner tube spacer has a proximal-side end face having the first outer facing surface.

5. The image diagnosis catheter according to claim 2, wherein
the transition surface and the first outer facing surface define a first flow path,
the second inner circumferential surface and the second outer facing surface define a second flow path,
the proximal-side end face of the inner tube and the third outer facing surface define a third flow path, and
the inner-tube-spacer partition flow path includes the first flow path, the second flow path, and the third flow path in this order from the upstream side to the downstream side.

6. The image diagnosis catheter according to claim 5, wherein
the first outer facing surface has a recess that defines the first flow path,
the second outer facing surface has a recess or a chamfered portion that defines the second flow path, and
the third outer facing surface has a recess that defines the third flow path.

7. The image diagnosis catheter according to claim 1, wherein
the inner tube spacer has a first inner facing surface that faces an outer circumferential surface of the inner-tube support tube and a second inner facing surface that faces a proximal-side end face of the inner-tube support tube.

8. The image diagnosis catheter according to claim 1, wherein
the inner tube spacer has an adhesive introduction path extending inside the inner tube spacer from an inlet port opened in the second outer facing surface to an outer circumferential surface of the inner-tube support tube between the first outer facing surface and the third outer facing surface.

9. The image diagnosis catheter according to claim 1, wherein
the second outer facing surface of the inner tube spacer is rotationally symmetric in a predetermined transverse plane perpendicular to a longitudinal direction of the inner tube.

10. The image diagnosis catheter according to claim 1, further comprising:
an outer-tube support tube provided on the radially inner side with respect to the outer tube and on the radially outer side with respect to the drive shaft;
an outer tube spacer that holds the outer-tube support tube so as to define an outer-tube support tube outer flow path;
a relay connector movable to the distal end side and the proximal end side relative to the inner tube together with the outer tube, the outer-tube support tube, and the outer tube spacer; and
an outer-tube-spacer distal-side flow path formed on the distal end side relative to the outer tube spacer,
wherein
at least the outer tube spacer defines an outer-tube-spacer partition flow path,
the inner flow path includes an outer-tube support tube inner flow path and the outer-tube-spacer distal-side flow path in this order from the upstream side to the downstream side, and
the outer flow path includes the outer-tube support tube outer flow path, the outer-tube-spacer partition flow path, and the outer-tube-spacer distal-side flow path in this order from the upstream side to the downstream side.

11. The image diagnosis catheter according to claim 10, wherein
the relay connector has a relay-connector first inner circumferential surface, a relay-connector second inner circumferential surface that is provided on the radially inner side with respect to the relay-connector first inner circumferential surface and that defines the outer-tube-spacer distal-side flow path, and a relay-connector transition surface that extends from the relay-connector first inner circumferential surface to the relay-connector second inner circumferential surface, and
the outer tube spacer has a first facing surface that faces an inner circumferential surface of the outer tube at a distal end portion, a second facing surface that faces a distal-side end face of the outer tube, a third facing surface that faces the relay-connector first inner circumferential surface, and a fourth facing surface that faces the relay-connector transition surface.

12. The image diagnosis catheter according to claim 11, wherein
the inner circumferential surface of the outer tube at the distal end portion and the outer tube spacer define an outer-tube-spacer first flow path,
the distal-side end face of the outer tube and the second facing surface define an outer-tube-spacer second flow path,
the relay-connector first inner circumferential surface and the third facing surface define an outer-tube-spacer third flow path,
the relay-connector transition surface and the fourth facing surface define an outer-tube-spacer fourth flow path, and
the outer-tube-spacer partition flow path includes the outer-tube-spacer first flow path, the outer-tube-spacer second flow path, the outer-tube-spacer third flow path, and the outer-tube-spacer fourth flow path in this order from the upstream side to the downstream side.

13. The image diagnosis catheter according to claim 12, wherein
the first facing surface has a recess or a chamfered portion that defines the outer-tube-spacer first flow path,
the second facing surface has a recess that defines the outer-tube-spacer second flow path,
the third facing surface has a recess or a chamfered portion that defines the outer-tube-spacer third flow path, and
the fourth facing surface has a recess that defines the outer-tube-spacer fourth flow path.

14. The image diagnosis catheter according to claim 10, wherein
the outer-tube support tube is provided on the radially inner side with respect to the inner tube and on the radially outer side with respect to the inner-tube support tube, and
in a most extended state in which the inner tube is maximally drawn out of the outer tube, a proximal-side end face of the outer-tube support tube is located distal to a distal-side end face of the inner tube, and a clearance is formed between an inner circumferential surface of the outer-tube support tube at a proximal end portion and an outer circumferential surface of the inner-tube support tube at a distal end portion.
